# EUROPEAN PATENT APPLICATION

(11) **EP 2 308 487 A1**
(43) Date of publication of application: **13.04.2011**
(21) Application number: 10011858.7
(22) Date of filing: 07.06.2006
(51) Int. Cl.: A61K 31/275, A61K 31/195, A61K 9/28, A61K 9/48, A61K 9/16

(54) **An entacapone-containing oral dosage form**

(30) Priority: 08.06.2005 US 688334 P
(62) Divisional of application: 09000405.2
(71) Applicant: ORION CORPORATION, 02200 Espoo (FI)
(72) Inventor: Vahervuo, Kari, 002320 Espoo (FI)
(74) Representative: Sexton, Jane Helen

(57) **Abstract**

The present invention relates to an oral dosage form of entacapone and to methods for the preparation thereof.

## Description

### FIELD OF THE INVENTION

The present invention relates to an oral dosage form of entacapone and to the methods that may be used in the preparation thereof.

### BACKGROUND OF THE INVENTION

Entacapone, ((E)-2-cyano-3-(3,4-dihydroxy-5-nitrophenyl)-N-diethyl-2 propenamide), is a catechol-O-methyl transferase (COMT) inhibitor that is used in combination with levodopa and a dopa decarboxylase (DDC) inhibitor to treat Parkinson's disease (PD). It is commercially available as a stand-alone formulation under the trademarks Comtess® and Comtan® and under trademark Stalevo® as a fixed combination (levodopa:carbidopa:entacapone: 50 mg:12.5 mg:200 mg, 100 mg:25 mg:200 mg and 150 mg:37.5 mg:200 mg).

Special attention is required when managing the symptoms of late stage PD patients who experience severe on-off fluctuations. Doctors usually prescribe them the stand-alone dosage form due to its flexibility in dosing. Those patients, especially the ones having swallowing problems, would benefit if a smaller dosage form would be available.

EP 1 112 065 B discloses the stand-alone entacapone tablet currently on the market under the tradenames Comtess® and Comtan®, and EP 1 189 608 B discloses the fixed combination product marketed under the trademark Stalevo®.

### SUMMARY OF THE INVENTION

Applicants have discovered that it is possible to make an oral dosage form of entacapone that is more easily swallowed while maintaining the oral bioavailability of entacapone.

It is one aspect of the invention to provide an oral dosage form comprising a pharmacologically effective amount of entacapone as the sole drug substance, wherein the amount of entacapone in a portion of the oral dosage form is from 35 % to 99 %, for instance from 48 % to 85 %, for instance from 50 % to 65 by weight of the portion.

It is a further aspect of the invention to provide a tablet comprising a pharmacologically effective amount of entacapone as the sole drug substance.

It is a further aspect of the invention to provide a core tablet comprising a pharmacologically effective amount of entacapone as the sole drug substance.

It is a further aspect of the invention to provide granules comprising a pharmacologically effective amount of entacapone as the sole drug substance.

It is a further aspect of the invention to provide a granule mixture comprising granules having a pharmacologically effective amount of entacapone as the sole drug substance, and one or more extragranular excipients.

It is a further aspect of the invention to provide a capsule comprising a pharmacologically effective amount of entacapone as the sole drug substance.

It is a further aspect of the invention to provide a kit comprising the dosage form according to the invention in combination with a dopamine precursor, such as levodopa, and a dopa decarboxylase inhibitor, for example carbidopa or benserazide, optionally with other drug substances, for example MAO B inhibitors.

It is a further aspect of the invention to provide a method of treating any condition wherein COMT inhibitors are found to be useful, particularly conditions wherein levodopa potentiation is needed, for example Parkinson's disease and restless legs syndrome (RLS, a condition characterized by an irresistible urge to move the legs, accompanied by other unpleasant sensations deep within the legs).

Additional aspects and advantages of the invention will be set forth in part in the description which follows, and in part will be obvious from the description, or may be learned by the practice of the invention. The objects and the advantages of the invention will be realized and attained by means of the elements and combinations particularly pointed out in the appended claims.

It is to be understood that both the foregoing general description and the following detailed description are exemplary and explanatory only and do not restrict the invention, as claimed.

### BRIEF DESCRIPTION OF THE DRAWINGS

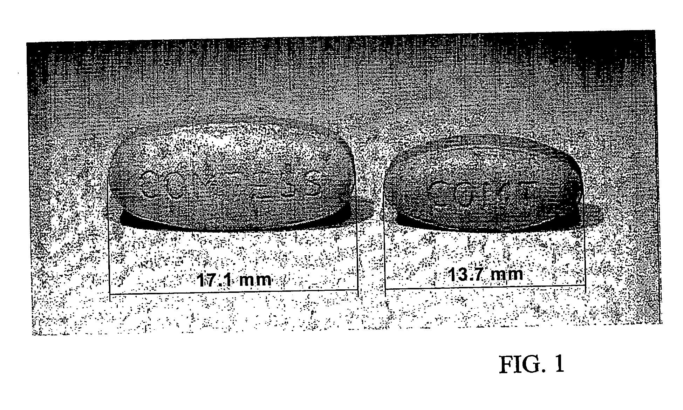

Fig. 1 shows the photographs of the present stand-alone tablet on the market (sold under the trademarks Comtess® and Comtan®) and a tablet according to the invention, respectively.

### DETAILED DESCRIPTION OF THE INVENTION

The invention relates to a new dosage form of entacapone that not only satisfies the requirements of the patients (such as those who have symptoms of late stage Parkinson's Disease with severe on-off fluctuations and patients having difficulty with swallowing), but also allows simple and cost-effective manufacturing without compromising the technical quality of the product.

The preparation of pharmaceutical compositions of entacapone has proved to be problematic. Entacapone is quickly absorbed in the gastrointestinal tract, however, the water solubility of entacapone is very low. Due to the poor solubility, the dissolution rate of entacapone can be a limiting factor for the absorption of entacapone in the gastrointestinal tract. The applicants have found that, in order to facilitate the absorption to the patient, entacapone having a certain reduced particle size as discussed below is preferably used in the dosage forms of the invention.

Entacapone particles of reduced particle size, however, have tended to cause manufacturing problems, thereby bringing about unnecessary delays in production. Potential problems related to reduced particle size include poor flowability and agglomeration which may result in compromised content uniformity (i.e. a reproducible amount of entacapone between different units).

As a solution to this problem, an oral dosage form of entacapone as well as methods for the preparation thereof has been invented by the applicants, which reduces or avoids the manufacturing problems such as those described above and improves the content uniformity while reducing the size of the final dosage form.

In one embodiment of the invention an oral dosage form is provided comprising a pharmacologically effective amount of entacapone as the sole drug substance, wherein the amount of entacapone in a portion of the oral dosage form is from 35 % to 99 % by weight of the portion.

In one embodiment of the invention an oral dosage form is provided comprising a pharmacologically effective amount of entacapone as the sole drug substance in a portion of the oral dosage form from 48 % to 85 % by weight of the portion.

In one embodiment of the invention an oral dosage form is provided comprising a pharmacologically effective amount of entacapone as the sole drug substance in a portion of the oral dosage form from 50 % to 65 % by weight of the portion.

The term "portion" of a dosage form can represent, for example, the dosage form excluding any outer coating made of pharmaceutically acceptable excipients. The term "portion" of a dosage form can also represent, for example, the dosage form excluding any capsule shell that contains the remainder of the dosage form.

Thus, where the dosage form is a core tablet, a "portion" of the dosage form can represent, for example, the entire dosage form. Where the dosage form is a tablet comprising a core tablet coated with pharmaceutically acceptable excipients, a "portion" of the dosage form can represent, for example, the tablet excluding the coating made of pharmaceutically acceptable excipients. Where the dosage form is a capsule, a "portion" of the dosage form can represent, for example, the contents enclosed by the capsule shell.

Reference to entacapone as "the sole drug substance" indicates that no other substance is present in a pharmacologically effective amount.

In one embodiment of the invention an oral dosage form is provided comprising a pharmacologically effective amount of entacapone as the sole drug substance, wherein entacapone is in particulate form, at least 90% of entacapone particles having a diameter less than 55 µm, for instance at least 90 % of particles having a diameter less than 35 µm. The particle size distribution by volume is measured using Laser Diffraction Particle sizing (LPD) by adding 1 mg of entacapone and 5 ml of hexadecane into a 5 ml beaker in ambient conditions and dispensing in an ultrasonic bath (Decon FS300, Decon Laboratories Ltd. U.K.) for 60 seconds and measuring the sample using the Malvern droplet and particle size analyser (2600C, model 2805LO, Malvern Instruments Ltd., U.K.) having a lens with the focal length of 63 mm. The percent values are calculated from the mean cumulative volume size curve using the Malvern 2600 software version B.22.

In one embodiment of the invention at least 90 % of entacapone particles have a diameter less than 35 µm.

In one embodiment of the invention not more than 20 % of entacapone particles have a diameter less than 2 µm.

In one embodiment of the invention an oral dosage form comprising entacapone as the sole drug substance is provided allowing at least 50 % of entacapone to be released within the first 30 minutes in a dissolution test (Apparatus 2 (USP), paddles at 50 rpm, pH 5.5) from said dosage form.

In one embodiment of the invention an oral dosage form comprising entacapone as sole drug substance is provided allowing at least 60 % of entacapone to be released within the first 45 minutes in a dissolution test (Apparatus 2 (USP), paddles at 50 rpm, pH 5.5) from said dosage form.

The oral dosage form according to the invention may be a tablet, a core tablet, a capsule or the like.

In one embodiment the dosage form of the invention comprises a binder. The binder may be, for instance one or more of following: acacia, alginic acid (Kelacid, Protacid, Satialgine H8), carbomer (Acritamer, Carbopol, Pemulen, Ultrez), carboxymethylcellulose sodium (Akucell, Aquasorb, Blanose, Finnfix, Nymcel, Tylose), ceratonia (Meyproffeur), cottonseed oil, dextrin (Avedex, Caloreen, Crystal Gum, Primogran W), dextrose (Caridex, Dextrofin, Lycedex PF, Roferose, Tabfine D-100), gelatin (Cryogel, Instagel, Solugel), guar gum (Galactosol, Meprogat, Meyprodor, Meyprofin, Meyproguar), hydrogenated vegetable oil type I (Akofine, Lubritab, Sterotex, Dynasan P60, Softisan 154, Hydrocote, Lipovol,HS-K, Sterotex HM), hydroxyethyl cellulose (Alcoramnosan, Cellosize, Idrorarnnosan, Liporamnosan, Natrosol, Tylose PHA), hydroxyethylmethyl cellulose (Culminal, Tylopur MH, Tylopur MHB, Tylose, MB, Tylose MH, Tylose MHB), hydroxypropyl cellulose (Klucel, Methocel, Nisso HPC), low substituted hydroxypropyl cellulose, hypromellose (Benecel MHPC, Methocel, Metolose, Pharmacoat, Spectracel 6, Spectracel 15, Tylopur), magnesium aluminium silicate (Carrisorb, Gelsorb, Magnabite, Neusilin, Pharmsorb, Veegum), maltodextrin (C*Dry,MD, Glucidex, Glucodry, Lycatab DSH, Maldex, Maltagran, Maltrin, Maltrin QD, Paselli MD 10 PH, Star-Dri) maltose (Advantose 100), methylcellulose (Benecel, Culminal MC, Methocel, Metolose), microcrystalline cellulose (Avicel PH, Celex, Celphere, Ceolus KG, Emcocel, Ethispheres, Fibrocel, Pharmacel, Tabulose, Vivapur), polydextrose (Litesse), polyethylene oxide (Polyox), polymethacrylates (Eastacryl 30D, Eudragit, Kollicoat MAE 30D, Kollicoat MAE 30DP), povidone (Kollidon, Plasdone), sodium alginate (Kelcosol, Keltone, Protanal), starch (Aytex P, Fluftex W, Instant Pure-Cote, Melojel, Meritena Paygel 55, Perfectamyl D6PH, Pure-Bind, Pure-Cote, Pure-Dent, Pure-Gel, Pure-Set, Purity 21, Purity 826, Tablet White) , pregelatined starch (Instastarch, Lycatab C, Lycatab PGS, Merigel, National 78-1551, Pharma-Gel, Prejel, Sepistab ST 200, Spress B820, Starch 1500 G, Tablitz, Unipure LD, Unipure WG 220), stearic acid (Crodacid, Emersol Hystrene, Industrene, Kortacid 1895, Pristerene), sucrose and zein.

In one embodiment of the invention the amount of the binder in a portion of the oral dosage form is from 0.5 % to 64.5 %, for instance from 1 % to 35 % by weight of the portion.

In one embodiment the binder is one or more of povidone, hypromellose, hydroxypropyl cellulose, methylcellulose and gelatine.

In one embodiment the binder is povidone. In one embodiment the binder is povidone and the amount of the binder in a portion of the oral dosage form is from 0.5 % to 8 %, for instance from 3 % to 6 % by weight of the portion.

In one embodiment of the invention the binder is a binder suitable for use in hot melt granulation (hot melt binder). Such a binder may be, for example one or more of the following: Polyethylene glycol (Breox PEG, Carbowax, Hodag PEG, Lutrol E), Stearic acid, Paraffin, Castor oil, hydrogenated (Castorwax, Castorwax MP 70, Castorwax MP 80, Opalwax, Sinulsol), Carnauba wax, Candelilla wax, Cottonseed oil, hydrogenated (Lubritab, Sterotex), glyceryl monostearate (Advawax 140, Atmul 67, Citomulgin M, Estol 603, Hodag GMS, Myvaplex 600P), acetylated glycerol monostearate, sorbitan monostearate (Capmul S, Liposorb S, Protachem SMS, Span 60), hexadecyl palmitate, octadecyl stearate, glyceryl trimyristate (Dynasan 114), glyceryl trilaurate (Dynasan 112), glyceryl tripalmitate (Dynasan 116), glyceryl tristearate (Dynasan 118) and glyceryl behenate (Compritol 888 Ato).

In one embodiment of the invention the hot melt binder is polyethylene glycol. In one embodiment of the invention the hot melt binder is polyethylene glycol and the amount of the hot melt binder in a portion of the oral dosage form is from 15% to 65 %, for instance from 25 % to 30 % by weight of the portion.

The dosage form according to the invention may further include, for instance one or more of disintegrants, fillers, solubility enhancers, glidants, lubricants (if applicable, for example, if tabletting) as well as other pharmaceutical excipients.

In one embodiment the dosage form according to the invention comprises a disintegrant. The disintegrant may be, for instance one or more of following: alginic acid (Kelacid, Protacid, Satialgine H8), calcium phosphate, tribasic (Tri-Cafos, TRI-CAL WG, TRI-TAB), carboxymethylcellulose calcium (ECG 505, Nymce1 ZSC), carboxymethylcellulose sodium (Akucell, Aquasorb, Blanose, Finnfix, Nymcel Tylose CB), colloidal silicon dioxide (Aerosil, Cab-O-Sil, Cab-O-Sil M-5P, Wacker HDK), croscarmellose sodium (Ac-Di-Sol, Explocel, Nymcel ZSX, Pharmacel XL, Primellose, Solutab, Vivasol), crospovidone (Kollidon CL, Kollidon CL-M, Polyplasdone XL, Polyplasdone XL-10), docusate sodium, guar gum (Galactosol, Meprogat, Meyprodor, Meyprofin, Meyproguar), low substituted hydroxypropyl cellulose, magnesium aluminun silicate (Carrisorb, Gelsorb, Magnabite, Neusilin, Pharmsorb, Veegum), methylcellulose (Benecel, Culminal MC, Methocel, Metolose), microcrystalline cellulose (Avicel PH, Celex, Celphere, Ceolus KG, Emcoel, Ethispheres, Fibrocel, Pharmacel, Tabulose, Vivapur), povidone (Kollidon, Plasdone) sodium alginate (Kelcosol, Keltone, Protanal), sodium starch glycolate (Explotab, Primojel, Vivastar P), polacrilin potassium (Amberlite IRP88), silicified microcrystalline cellulose (ProSolv), starch (Aytex P, Fluftex W, Instant Pure-Cote, Melojel, Meritena, Paygel 55, Perfectamyl D6PH, Pure-Bind, Pure-Cote, Pure-Dent, Pure-Gel, Pure-Set, Purity 21, Purity 826, Tablet White) or pre-gelatinezed starch (Instanstarch, Lycatab C, Lycatab PGS, Merigel, National 78-1551, Pharma-Gel, Prejel, Sepistab ST 200, Spress B820, Starch 1500 G, Tablitz, Unipure LD and Unipure WG220).

In one embodiment of the invention the amount of the disintegrant in a portion of the oral dosage form is from 0.5 % to 64.5 %, for instance from 2 % to 40 % by weight of the portion.

In one embodiment the disintegrant is one or more of croscarmellose sodium, crospovidone, low substituted hydroxypropyl cellulose, microcrystalline cellulose and sodium starch glycolate.

In one embodiment the disintegrant is croscarmellose sodium. In one embodiment the disintegrant is croscarmellose sodium and the amount of the disintegrant in a portion of the oral dosage form is from is 0.5 % to 35 %, for instance from 18 % to 27 %, for instance from 20 % to 25 % by weight of the portion.

In one embodiment, the disintegrant is microcrystalline cellulose. In one embodiment, the disintegrant is microcrystalline cellulose and the amount of microcrystalline cellulose in a portion of the oral dosage form is from 0.5 % to 64.0 %, for instance from 5 % to 20 % by weight of the portion.

In one embodiment the disintegrant comprises croscarmellose sodium and microcrystalline cellulose, and the proportion of croscarmellose sodium to microcrystalline cellulose is from 0.5:99.5 to 99.5:0.5 by weight, for instance from 40:60 to 80:20 by weight in a portion of the oral dosage form.

In one embodiment the disintegrant is low substituted hydroxypropyl cellulose (L-HPC). In one embodiment the disintegrant is low substituted hydroxypropyl cellulose and the amount of the disintegrant in a portion of the oral dosage form is from is 0.5 % to 40 %, for instance from 15 % to 35 % by weight of the portion.

In one embodiment the dosage form according to the invention comprises a solubility enhancer. The solubility enhancer may be, for instance one or more of the following: cyclodextrins (Cavitron, Encapsin, Rhodocap, Kleptose), glyceryl monostearate (Abracol SLG, Admul, Myvaplex 600P), lecithin, poloxamer (Lutrol, Monolan, Pluronic), polyoxyethylene fatty acid esters (polysorbates) (Tween), docusate sodium (Cropol), sodium lauryl sulphate (Elfan 240, Maprofix 563), sorbitan esters (sorbitan fatty acid esters) (Span), polyvinyl pyrrolidone, polyethylene glycol (PEG), lauryl macrogol glyceride (Gelucire) and d-alpha-tocophenyl PEG succinate (Vitamin E TPGS NF).

In one embodiment the solubility enhancer is one or more of polyethylene glycol, polyvinyl pyrrolidone, lauryl macrogol glyceride and d-alpha-tocophenyl PEG succinate.

In one embodiment the dosage form according to the invention comprises a filler which may be used e.g. as a processing aid. The filler may be, for instance one or more of calcium carbonate (Barcroft, Cal-Carb, CalciPure, Destab, MagGran, Millicarb, Pharma-Carb, Precarb, Sturcal, Vivapres Ca), calcium phosphate, dibasic anhydrous (A-TAB, Di-Cafos A-N, Emcompress Anhydrous, Fujicalin), calcium phosphate, dibasic dihydrate (Cafos, Calipharm, Calstar, Di-Cafos, Emcompress), calcium phosphate tribasic (Tri-Cafos, TRI-CAL WG, TRI-TAB), calcium sulphate (Destab, Drierite, Snow White, Cal-Tab, Compactrol, USG Terra Alba), cellulose powdered (Arbocel, Elcema, Sanacel, Solka-Floc), silicified microcrystalline cellulose (ProSolv), cellulose acetate, compressible sugar (Di-Pac), confectioner's sugar, dextranes (Candex, Emdex), dextrin (Avedex, Caloreen, Crystal Gum, Primogran W), dextrose (Caridex, Dextrofin, Lycadex PF, Roferose, Tabfine D-100), fructose (Advantose, Fructamyl, Fructofin, Krystar), kaolinLion, Sim 90), lactitol (Finlac ACX, Finlac DC, Finlac MCX), lactose (Aero Flo 20, Aero Flo 65, Anhydrox, CapsuLac, Fast-Flo, FlowLac, GranuLac, InhaLac, Lactochem, Lactohale, Lactopress, Microfine, Microtose, Pharmatose, Prisma Lac, Respitose, SacheLac, SorboLac, Super-Tab, Tablettose, Wyndale, Zeparox), magnesium carbonate, magnesium oxide (MagGran MO), maltodextrin (C*Dry MD, Glucidex, Glucodry, Lycatab DSH, Maldex, Maltagran, Maltrin, Maltrin QD, Paselli MD 10 PH, Star-Dri), maltose (Advantose 100), mannitol (Mannogem, Pearlitol), microcrystalline cellulose (Avicel PH, Celex, Celphere, Ceolus KG, Emcocel, Ethispheres, Fibrocel, Pharmacel, Tabulose, Vivapur), polydextrose (Litesse), simethicone (Dow Coming Q7-2243 LVA, Cow Coming Q7-2587, Sentry Simethicone), sodium alginate (Kelcosol, Keltone, Protanal), sodium chloride (Alberger), sorbitol (Liponec 70-NC, Liponic 76-NC, Meritol, Neosorb, Sorbifin, Sorbitol Instant, Sorbogem), starch (Aytex P, Fluftex W, Instant Pure-Cote, Melojel, Meritena Paygel 55, Perfectamyl D6PH, Pure-Bind, Pure-Cote, Pure-Dent, Pure-Gel, Pure-Set, Purity 21, Purity 826, Tablet White), pregelatinized starch (Instastarch, Lycatab C, Lycatab PGS, Merigel, National 78-1551, Pharma-Gel, Prejel, Sepistab ST 200, Spress B820, Starch 1500 G, Tablitz, Unipure LD, Unipure WG220), sucrose, trehalose and xylitol (Klinit, Xylifin, Xylitab, Xylisorb, Xylitolo).

In one embodiment the filler is one or more of silicified microcrystalline cellulose, microcrystalline cellulose and pregelatinized starch.

In one embodiment the dosage form according to the invention comprises a glidant. The glidant may be, for instance one or more of the following: tribasic calcium phosphate (Tri-Cafos, TRI-CAL, TRI-TAB), calcium silicate, cellulose, powdered (Arbocel, Elcema, Sanacel, Solka-Floc), colloidal silicon dioxide (Aerosil, Cab-O-Sil, Cab-O-Sil M-5P, Wacker HDK), magnesium silicate, magnesium trisilicate, starch Aytex P, Fluftex W, Instant Pure-Cote, Melojel, Meritena, Paygel 55, Perfectamyl D6PH, Pure-Bind, Pure-Cote, Pure-Dent, Pure-Gel, Pure-Set, Purity 21, Purity 826, Tablet White and talc (Altalc, Luzenac, Luzenac Pharma, Magsil Osmanthus, Magsil Star, Superiore).

In one embodiment the glidant is colloidal silicon dioxide and/or talc.

In case the dosage form is a tablet or a core tablet a lubricant may be used to improve the tabletting. The lubricant may be for instance one or more of following: calcium stearate (HyQual), glycerine monostearate (Capmul GMS-50, Cutina GMS, Imwitor 191 and 900, Kessco GMS, Lipo GMS 410, 450 and 600, Myvaplex 600P, Myvatex, Protachem GMS-450, Rita GMS, Stepan GMS, Tegin, Tegin 503 and 515, Tegin 4100, Tegin M, Unimate GMS), glyceryl behenate (Compritol 888 ATO), glyceryl palmitostearate Precirol ATO 5), hydrogenated castor oil (Castorwax, Castorwax MP 70, Castorwax MP 80, Croduret, Cutina HR, Fancol, Simulsol 1293), hydrogenated vegetable oil type I (Akofine, Lubritab, Sterotex, Dynasan P60, Softisan 154, Hydrocote, Lipovol HS-K, Sterotex HM), magnesium lauryl sulphate, magnesium stearate, medium-chain triglycerides (Captex 300, Captex 355, Crodamol GTC/C, Labrafac CC, Miglyol 810, Miglyol 812, Myritol, Neobee M5, Nesatol, Waglinol 3/9280), poloxamer (Lutrol, Monolan, Pluronic, Supronicm Synperonic), polyethylene glycol (Carbowax, Carbowax Sentry, Lipo, Lipoxol, Lutrol E, Pluriol E), sodium benzoate (Antimol), sodium chloride (Alberger), sodium lauryl sulphate (Elfan 240, Texapon K12P), sodium stearyl fumarate (Pruv), stearic acid (Crodacid E570, Emersol, Hystrene, Industrene, Kortacid 1895, Pristerene), talc (Altalc, Luzenac, Luzenac Pharma, Magsil Osmanthus, Magsil Star, Superiore), sucrose stearate (Surfhope SE Pharma D-1803 F and zinc stearate (HyQual).

In one embodiment, the lubricant is one or more of glyceryl behenate, magnesium stearate, sodium lauryl sulphate and sucrose stearate.

In one embodiment the lubricant is magnesium stearate. In one embodiment the lubricant is magnesium stearate and the amount magnesium stearate in a portion of the oral dosage form is from 0.1 % to 3 % by weight of the portion.

In one embodiment of the invention the dosage form is a coated tablet. In one embodiment of the invention the tablet is coated, for instance with a cellulose derivative or polyvinyl alcohol based coating, polyethylene glycols, acrylate polymers or sugar coating or mixtures thereof. Preferably, a water based coating is used.

In one embodiment of the invention the coating comprises dyes, colour lakes, or pigments, such as iron oxides, for instance yellow or red irons oxides and titanium dioxide.

In one embodiment of the invention the dosage form is a coated tablet and the weight of the tablet coating is from 0.5 to 10 % compared with the weight of the core tablet, for instance from 1 to 4 % compared with the weight of the core tablet.

In one embodiment of the invention an oral dosage form is provided comprising entacapone as the sole drug substance and a disintegrant, wherein the amount of entacapone in a portion of the oral dosage form is from 35 % to 99 %, for example from 48 % to 85 %, for example from 50 % to 65 % by weight of the portion and the amount of the disintegrant is from 0.5 % to 64.5 %, for example from 2 % to 40 % by weight of the portion.

In one embodiment of the invention an oral dosage form is provided comprising entacapone as the sole drug substance, a disintegrant and a binder, wherein the amount of entacapone in a portion of the oral dosage form is from 35 % to 99 %, for example from 48 % to 85 %, for example from 50 % to 65 % by weight of the portion and the amount of the disintegrant is from 0.5 % to 64.5 %, for example from 2 % to 40 % by weight of the portion and the amount of the binder is from 0.5 % to 64.5 %, for instance from 1 % to 35 % by weight of the portion.

In one embodiment of the invention an oral dosage form is provided comprising entacapone as the sole drug substance and croscarmellose sodium, wherein the amount of entacapone in a portion of the oral dosage form is from 35 % to 99 %, for example from 48 % to 85 %, for example from 50 % to 65 % by weight of the portion and the amount of croscarmellose sodium is from 0.5 % to 35 %, for example from 18 % to 27 %, for example from 20 % to 25 % by weight of the portion.

In one embodiment of the invention an oral dosage form is provided comprising entacapone as the sole drug substance, croscarmellose sodium and povidone, wherein the amount of entacapone in a portion of the oral dosage form is from 35 % to 99 %, for example from 48 % to 85 %, for example from 50 % to 65 % by weight of the portion, the amount of croscarmellose sodium is from 0.5 % to 35 %, for example from 18 % to 27 %, for example from 20 % to 25 % by weight of the portion and the amount of povidone is from 0.5 % to 8%, for example from 3 % to 6 % by weight of the portion.

In one embodiment of the invention an oral dosage form is provided comprising entacapone as the sole drug substance, croscarmellose sodium, microcrystalline cellulose and povidone, wherein the amount of entacapone in a portion of the oral dosage form is from 35 % to 99 %, for example from 48 % to 85 %, for example from 50 % to 65 % by weight of the portion, the amount of croscarmellose sodium is from 0.5 % to 35 %, for example from 18 % to 27 %, for example from 20 % to 25 % by weight of the portion, the amount of microcrystalline cellulose is from 0.5 % to 64.0 %, for instance from 5 % to 20 % by weight of the portion and the amount of povidone is from 0.5 % to 8%, for example from 3 % to 6 % by weight of the portion.

In one embodiment of the invention a tablet comprising 200 mg of entacapone as the sole drug substance is provided having the following dimensions when measured at the outermost dimensions of the tablet:
length from 11 mm to 16 mm, for instance from 13 mm to 15 mm
width from 4 mm to 9 mm, for instance from 6 mm to 8 mm
height from 4 mm to 7 mm, for instance from 4 mm to 6 mm.

In one embodiment of the invention the tablet is oval. In another embodiment of the invention the tablet is round.

In one embodiment of the invention a tablet is provided comprising entacapone as the sole drug substance and croscarmellose sodium, wherein the amount of entacapone in a portion of the oral dosage form is from 35 % to 99 %, for example from 48 % to 85 %, for example from 50 % to 65 % by weight of the portion and the amount of croscarmellose sodium is from 0.5 % to 35 %, for example from 18 % to 27 %, for example from 20 % to 25 % by weight of the portion.

In one embodiment of the invention a tablet is provided comprising entacapone as the sole drug substance, croscarmellose sodium and povidone, wherein the amount of entacapone in a portion of the oral dosage form is from 35 % to 99 %, for example from 48 % to 85 %, for example from 50 % to 65 % by weight of the portion, the amount of croscarmellose sodium is from 0.5 % to 35 %, for example from 18 % to 27 %, for example from 20 % to 25 % by weight of the portion and the amount of povidone is from 0.5 % to 8 %, for example from 3 % to 6 % by weight of the portion.

In one embodiment of the invention a tablet is provided comprising entacapone as the sole drug substance, croscarmellose sodium, microcrystalline cellulose and povidone, wherein the amount of entacapone in a portion of the oral dosage form is tom 35 % to 99 %, for example from 48 % to 85 %, for example from 50 % to 65 % by weight of the portion, the amount of croscarmellose sodium is from 0.5 % to 35 %, for example from 18 % to 27 %, for example from 20 % to 25 % by weight of the portion, the amount of microcrystalline cellulose is from 0.5 % to 64.0 %, for instance from 5 % to 20 % by weight of the portion and the amount of povidone is from 0.5 % to 8 %, for example from 3 % to 6 % by weight of the portion.

In one embodiment of the invention a tablet is provided comprising entacapone as the sole drug substance, croscarmellose sodium, microcrystalline cellulose, povidone and magnesium stearate, wherein the amount of entacapone in a portion of the oral dosage form is from 35 % to 99 %, for example from 48 % to 85 %, for example from 50 % to 65 % by weight of the portion, the amount of croscarmellose sodium is from 0.5 % to 35 %, for example from 18 % to 27 %, for example from 20 % to 25 % by weight of the portion, the amount of microcrystalline cellulose is from 0.5 % to 64.0 %, for instance from 5 % to 20 % by weight of the portion, the amount of povidone is from 0.5 % to 8 %, for example from 3 % to 6 % by weight of the portion and the amount of magnesium stearate is from 0.1 % to 3 % by weight of the portion.

In one embodiment of the invention a tablet is provided comprising entacapone as the sole drug substance, croscarmellose sodium, microcrystalline cellulose, portion of the oral dosage form is from 35 % to 99 %, for example from 48 % to 85 %, for example from 50 % to 65 % by weight of the portion, the amount of croscarmellose sodium is from 0.5 % to 35 %, for example from 18 % to 27 %, for example from 20 % to 25 % by weight of the portion, the amount of microcrystalline cellulose is from 0.5 % to 64.0 %, for instance from 5 % to 20 % by weight of the portion, the amount of povidone is from 0.5 % to 8 %, for example from 3 % to 6 % by weight of the portion, the amount of magnesium stearate is from 0.1 % to 3 % by weight of the portion and the amount of the coating is from 0.1 % to 10 %, for example from 1 % to 4 % by weight of the total dosage form.

In one embodiment granules comprising a pharmacologically effective amount of entacapone as the sole drug substance are provided having the following granule size distribution by volume: the 10 % fractile is, for instance from 7 µm to 60 µm, for instance from 10 µm to 40 µm and the 90 % fractile is, for instance from 600 µm to 1200 µm, for instance from 750 µm to 1100 µm and the median is, for instance from 80 µm to 600 µm, for instance from 100 µm to 300 µm. The granule size distribution by volume is measured using laser diffraction with dry dispersion (Laser Diffraction Particle Size Analyzer LS13320, Beckman Coulter Inc., USA). The analysis is carried out in ambient conditions. The samples are dispersed in air using Tornado Dry Powder System. The dispersion pressure is 19 inches H2O and the sample amount is 20 ml. The percent values are calculated from the mean cumulative volume size curve using the Beckman Coulter LS13320 software version 4.21.

In one embodiment of the invention granules are provided having a water activity from 0.10 to 0.65, for instance from 0.2 to 0.4 (measured by the equipment sold under the tradename Aqualab®).

In one embodiment a granule mixture is provided comprising the granules described herein above and one or more extragranular excipients.

In one embodiment a hard gelatine capsule is provided comprising the granules or the granule mixture according to the invention.

In one embodiment of the invention the hard gelatine capsule comprising the granules or the granule mixture has a capsule shell size from 2 to 00, for example from 1 to 0.

In one embodiment of the invention a kit is provided comprising the dosage form according to the invention in combination with a dopamine precursor, such as levodopa, and a dopa decarboxylase inhibitor, for example carbidopa or benserazide, optionally with other drug substances, for example MAO B inhibitors. The dosage forms in the kit may be provided for sequential or simultaneous administration.

In one embodiment of the invention a core tablet is prepared by (i) preparing a mixture of entacapone as the sole drug substance with a binder and optionally one or more other excipients and granulating with a granulating liquid; or granulating entacapone together with one or more optional other excipients with a granulating liquid comprising a binder or by melt granulation; (ii) drying or cooling, respectively and optionally screening the granules of step (i), optionally bringing the granules into contact with one or more extragranular excipients and (iii) compressing the granules and the optional extragranular excipients into a core tablet.

It is typical to divide the granulation in three steps: 1) premixing step 2) addition of the granulation liquid and 3) the so called kneading step. In many cases, steps 2 and 3 are combined. However, it is to be understood that it is also possible to carry out all three steps concurrently.

In one embodiment of the invention the preparation of the mixture of entacapone and the binder and / or optional other excipients is conducted, for instance by diffusion blending (tumble), convection mixing, for example in a high shear mixer or a single pot processor, or using continuous granulation, for example in a twin screw mixer.

Process conditions for a commercial scale (1.0-1.5 m³) vertical high shear mixer during the premixing step may be the following: mixing time from 0.5 to 5.5 min, for instance from 1 to 3 min, impeller rate from 50 to 200 rpm, for instance from 80 to 120 rpm and chopper rate from 0 to 800 rpm.

In one embodiment of the invention the granulation step is carried out in e.g. in a high shear mixer, a single pot processor or by using continuous granulation.

Process conditions for a commercial scale vertical high shear mixer during the addition of water based granulation liquid may be the following: mixing time from 5 to 20 min, for example from 8 to 16 min., impeller rate from 50 to 200 rpm, for instance from 80 to 120 rpm and chopper rate from 0 to 800 rpm.

Process conditions for a commercial scale vertical high shear mixer during the kneading step may be the following: mixing time from 0.5 to 15 min, for example from 2 to 8 min., impeller rate from 50 to 200 rpm, for instance from 80 to 120 rpm and chopper rate from 0 to 800 rpm.

In one embodiment of the invention the proportion of the binder to the granulation mass is from 0.5:99.5 to 64.5:35.5, for example from 1:99 to 35:65 by dry weight.

In one embodiment of the invention the binder is one or more of povidone, hypromellose, hydroxypropyl cellulose, methylcellulose and gelatine.

In one embodiment of the invention the solvent used in the granulating liquid is water, a lower alcohol such as ethanol, isopropanol or a mixture thereof.

In one embodiment of the invention the granulation liquid is water. In one embodiment of the invention the granulation liquid is water and the disintegrant is croscarmellose sodium and the amount of water added during granulation is from 50 % to 120 %, for example from 70 % to 100 % compared with the dry weight of the granulation mass. It has been found that using said amounts of water improves flowability and compactibility.

In one embodiment of the invention the granulation liquid is water and the disintegrant is L-HPC and the amount of water added during granulation is from 30 % to 100 %, for example from 40 % to 80 % compared with the dry weight of the granulation mass.

In one embodiment of the invention the granulation liquid is a dispersion or suspension.

In one embodiment of the invention the granules are dried, for instance in a single pot processor, in a fluidized bed or in a drying chamber.

In one embodiment of the invention the granules are dried directly in the granulation equipment.

In one embodiment of the invention the granules are dried directly in the granulation equipment which is a single pot processor.

In one embodiment of the invention the granules are dried using the fluidized bed and the equipment is pre-heated before drying.

Process conditions for a commercial scale fluidized bed dryer (from 50 to 300 kg) are the following: inlet air temperature from 60 °C to 110 °C, for instance from 75 °C to 85 °C, outlet air temperature at the end of drying from 25 °C to 60 °C, for instance from 30 °C to 55 °C, air flow rate during drying from 1500 m³/h to 5000 m³/h, for instance from 2500 m³/h to 4500 m³/h, drying time from 20 min to 200 min, for instance from 50 min to 170 min.

The temperature of the liquid within the jacket of a commercial scale single pot processor (from 50 to 300 kg) may be from 30 °C to 80 °C, for instance from 35 °C to 60 °C. The drying time depends on the size of the equipment and on whether the drying is assisted, for instance by one or more of the following: vacuum, gas stripping, swinging bowl system and microwaves.

The inner temperature of a commercial scale a drying chamber may be from 30 °C to 80 °C, for instance from 35 °C to 55 °C. The drying time depends on the size of the equipment and on whether the drying is assisted.

In one embodiment of the invention the granulation is carried out by melt granulation.

In the case of melt granulation the granules are cooled after the granulation.

In one embodiment of the invention the granules are screened e.g. in a rotating impeller, a rotating screen or an oscillating bar screening mill.

In one embodiment granules are screened to the following granule size distribution by volume: the 10 % fractile is, for instance from 7 µm to 60 µm, for instance from 10 µm to 40 µm and the 90 % fractile is, for instance from 600 µm to 1200 µm, for instance from 750 µm to 1100 µm and the median is, for instance from 80 µm to 600 µm, for instance from 100 µm to 300 µm. The granule size distribution by volume is measured using laser diffraction with dry dispersion (Laser Diffraction Particle Size Analyzer LS13320, Beckman Coulter Inc., USA). The analysis is carried out in ambient conditions. The samples are dispersed in air using Tornado Dry Powder System. The dispersion pressure is 19 inches H2O and the sample amount is 20 ml. The percent values are calculated from the mean cumulative volume size curve using the Beckman Coulter LS13320 software version 4.21.

In one embodiment of the invention one or more extragranular excipients are brought into contact with the granules, for example by diffusion blending (tumble), convection mixing or pneumatic mixing.

In one embodiment of the invention instead of blending the granules with a lubricant the excipients facilitating the lubrication of the dies of the tablet press are sprayed directly on the surfaces of the dies.

In one embodiment of the invention the granules (together with optional extragranular excipients) are compressed into core tablets using a tablet press whose feeding is based on gravity.

In one embodiment of the invention the granules (together with optional extragranular excipients) are compressed into core tablets using a power assisted or centrifugal tablet press.

In one embodiment of the invention, the core tablets prepared by this method have the crushing strength from 70 N to and 220 N.

In one embodiment of the invention the core tablets are coated, for instance with a cellulose derivative or polyvinyl alcohol based coating, polyethylene glycols, acrylate polymers or sugar coating or mixtures thereof. Preferably, a water based coating is used.

In one embodiment of the invention the coating comprises dyes, colour lakes, or pigments, such as iron oxides, for instance yellow or red irons oxides, and titanium dioxide.

In one embodiment of the invention the increase of the weight of the tablet due to the application of the coating is, for instance from 0.5 % to 10.0 %, for instance from 1 % to 4 % by weight.

In one embodiment of the invention a core tablet is prepared by (i) preparing a mixture of entacapone as the sole drug substance with a binder and a disintegrant and optionally one or more other excipients and granulating with a granulating liquid (ii) drying and optionally screening the granules of step (i), optionally bringing the granules into contact with one or more extragranular excipients and (iii) compressing the granules and the optional extragranular excipients into a core tablet.

In one embodiment of the invention a core tablet is prepared by (i) preparing a mixture of entacapone as the sole drug substance with a binder and a disintegrant and optionally one or more other excipients and granulating with a granulating liquid (ii) drying and screening the granules of step (i), optionally bringing the granules into contact with one or more extragranular excipients and (iii) compressing the granules and the optional extragranular excipients into a core tablet.

In one embodiment of the invention a core tablet is prepared by (i) preparing a mixture of entacapone as the sole drug substance with povidone, croscarmellose sodium and optionally one or more other excipients and granulating with water (ii) drying and screening the granules of step (i), optionally bringing the granules into contact with one or more extragranular excipients and (iii) compressing the granules and the optional extragranular excipients into a core tablet.

In one embodiment of the invention a core tablet is prepared by (i) preparing a mixture of entacapone as the sole drug substance with povidone, croscarmellose sodium and optionally one or more other excipients and granulating with water, the amount of water added during granulation being from 50 % to 120 % compared with the dry weight of the granulation mass (ii) drying and screening the granules of step (i), optionally bringing the granules into contact with one or more extragranular excipients and (iii) compressing the granules and the optional extragranular excipients into a core tablet.

In one embodiment of the invention a core tablet is prepared by (i) preparing a mixture of entacapone as the sole drug substance with povidone, croscarmellose sodium microcrystalline cellulose; the proportion of croscarmellose sodium to microcrystalline cellulose being from 0.5:99.5 to 99.5:0.5 by weight; and optionally one or more other excipients and granulating with water (ii) drying and screening the granules of step (i), optionally bringing the granules into contact with one or more extragranular excipients and (iii) compressing the granules and the optional extragranular excipients into a core tablet.

In one embodiment of the invention a core tablet is prepared by (i) preparing a mixture of entacapone as the sole drug substance with povidone, croscarmellose sodium microcrystalline cellulose; the proportion of croscarmellose sodium to microcrystalline cellulose being from 0.5:99.5 to 99.5:0.5 by weight; and optionally one or more other excipients and granulating with water the amount of water added during granulation being from 50 % to 120 % compared with the dry weight of the granulation mass (ii) drying and screening the granules of step (i), optionally bringing the granules into contact with one or more extragranular excipients and (iii) compressing the granules and the optional extragranular excipients into a core tablet.

In one embodiment of the invention granules are prepared by (i) preparing a mixture of entacapone as the sole drug substance with a binder and optionally one or more other excipients and granulating the mixture with a granulating liquid or granulating entacapone together with one or more optional other excipients with a granulating liquid comprising a binder or by melt granulation; (ii) drying or cooling, respectively and optionally screening the granules of step (i).

As it comes to steps (i) and (ii) above, conditions, excipients and equipment etc. described in connection of the preparation of the core tablet may be used.

In another embodiment of the invention a granule mixture is prepared by (i) preparing a mixture of entacapone as the sole drug substance with a binder and optionally one or more other excipients and granulating the mixture with a granulating liquid or granulating entacapone together with one or more optional other excipients with a granulating liquid comprising a binder or by melt granulation; (ii) drying or cooling, respectively and optionally screening the granules of step (i), bringing the granules into contact with one or more extragranular excipients.

As it comes to steps (i) and (ii) above, conditions, excipients and equipment etc. described above in connection of the preparation of the core tablet may be used.

In one embodiment of the invention a capsule is prepared by (i) preparing a mixture of entacapone as the sole drug substance with a binder and optionally one or more other excipients and granulating the mixture with a granulating liquid or granulating entacapone together with one or more optional other excipients with a granulating liquid comprising a binder or by melt granulation; (ii) drying or cooling, respectively and optionally screening the granules of step and (i) optionally bringing the granules into contact with one or more extragranular excipients and (iii) filling the granules and optional extragranular excipients into a suitable capsule.

As it comes to steps (i) and (ii) above, conditions, excipients and equipment etc. described above in connection of the preparation of the core tablet may be used.

In one embodiment of the invention the size of a hard gelatine capsule is used having a shell size from 2 to 00, for instance from 1 to 0 in the preparation of the capsule. The weight of a capsule shell of this size is typically from 60 mg to 125 mg.

The term "cellulose derivative" used herein includes chemically modified cellulose wherein the carbon skeleton of the cellulose is unchanged, such as cellulose acetate, ethylcellulose (Aquacoat, Ethocel, Surelease), hydroxyethyl cellulose (Natrosol, Tylose PHA), hydroxyethylmethyl cellulose (Tylopur MH, Tylose MB), hydroxypropyl cellulose (Klucel, Methocel), hypromellose (Metolose, Pharmacoat), hypromellose phthalate or methylcellulose (Benecel, Methocel, Metolose).

The term "polyvinyl alcohol based coating" encompasses the tablet coating-films in which the polyvinyl alcohol (Airvol, Elvanol, Gohsenol) is used as the film-forming agent.

The term "cooling" encompasses active cooling or allowing cool to the ambient temperature.

The term "melt granulation" encompasses a size enlargement process where fine powder feed particles are bound together by a process where the binder is added as a solid and mixed with the powder while the granulator temperature is raised above the melting point of the binder or the melted binder is sprayed on the powder in a fluidized bed granulator.

The term "granule mixture" encompasses the mixture of the granules according to the invention with one or more extragranular excipients including granules and powders.

The term "fractionating" encompasses any conventional method which sorts the particles according to their size, such as sieving method or air fractionating method.

As a general textbook reference on providing more information of the excipients mentioned herein a reference is made to Handbook of Pharmaceutical Excipients, Ed. Rowe R. et al. Pharmaceutical Press, American Pharmaceutical Association, Fourth edition, 2003.

Entacapone may be prepared, for instance by the methods described US 5,446,194 and US 5,135,950, which are incorporated herein by reference. Entacapone may exist as any mixture of (E)- and (Z) isomers or in a form of a substantially pure (E)-or (Z) isomer. Preferably, entacapone is in a substantially pure (E)-isomeric form. The (E)-isomer may take different polymorphic forms, e.g. polymorph A disclosed in US 5,135,950 or polymorph D disclosed in patent application No. WO 2005/063696.

Also different salts having comparable dissolution properties to that of entacapone may be used instead of free entacapone, i.e. salts having an intrinsic dissolution rate comparable to entacapone in discriminative test conditions. Intrinsic dissolution rate is defined as dissolution rate of pure substances under the condition of constant surface area. The intrinsic dissolution rate is determined by exposing the constant surface area of material to an appropriate dissolution medium while maintaining constant temperature, stirring rate, and pH (phosphate buffer, 37 °C, 50 rpm, pH 5.5 in this case).

Entacapone (or its salt, respectively) having the particle size distribution defined above can be provided in several ways, for instance by reducing the particle size of entacapone particles obtained from the compound synthesis, for instance mechanically by milling (for example by a bal mill, a fluid energy attrition mill or a jet mil), by ultrasonic means and/ or by fractionating or by crystallizing it directly to the desired particle size.

As a general textbook reference on providing particles of desired size as well as to manufacturing technologies mentioned herein a reference is made to Remington's Pharmaceutical Sciences, 18th ed, 1990, Mack Publishing Company, Easton, Pennsylvania 18042. Suitable manufacturing technologies may be also found in Pharmaceutics the Science of Dosage Form Design Ed. M.E. Aulton, 2000.

In order to be effective, for example in the treatment of Parkinson's disease or Restless Legs Syndrome each dosage form according to the invention is to be taken sequentially or simultaneously with a dopamine precursor (such as levodopa or ethyl ester of levodopa) and a DDC inhibitor and optional other drug substances.

The pharmacologically effective amounts of entacapone, levodopa and the DDC inhibitor are dependent on numerous factors known to those skilled in the art, such as, the type and the severity of the condition of the patient, the highest recommended daily dose being 200 mg of entacapone ten times a day (i.e. 2000 mg entacapone per day). For example, in the treatment of RLS the daily dose of levodopa can be as low as 50 mg, for example from 50 mg to 300 mg but can be from 200 mg to 600 mg, divided into 1 to 4, preferable into 1 to 2 individual doses, whereas in the treatment of severely ill Parkinsonian patients the daily dose of levodopa can be considerably higher, for example from 100 mg to 2000 mg divided, for example from two to ten individual doses.

The amount of entacapone in a single dosage unit according to the invention is, for instance from 100 mg to 400 mg, e.g. from 100 mg to 300 mg, especially from 100 mg to 200 mg. The amount of levodopa in a single dosage unit is, for instance from 50 mg to 400 mg, e.g. from 50 mg to 300 mg, for example from 50 mg to 200 mg.

DDC inhibitors include, without limitation, carbidopa and benserazide. Levodopa and carbidopa are commercially available both as immediate release and slow release (depot) combination tablets sold in Europe under, for instance, the following trademarks: Nacom®, Sinemet®, Sinemet Depot® and Sinemet® Plus. Levodopa and benserazide are commercially available both as immediate release and slow release (depot) combination tablets, for instance, under the trademark Madopar® and Rextex®.

The amount of carbidopa in a single dosage unit is, for instance from 5 mg to 200 mg, e.g. from 5 mg to 100 mg, e.g. from 5 mg to 50 mg.

DDC inhibitor and levodopa (or other dopamine precursor) are administered, for example in a ratio of from 1:1 to 1:40, for example from 1:4 to 1:10.

The MAO B inhibitors include, without limitation selegiline, rasagiline, lazabemide and safinamide. The daily dose of selegiline is from 1 to 20 mg, for example from 2 to 10 mg, divided into 1 to 10, preferably into 1 to 2 individual doses. The daily dose of rasagiline is from 0.1 to 5.0 mg, for instance from 0.5 to 2 mg divided into 1 to 10, preferably into 1 to 2 individual doses. The daily dose of lazabemide is from 100 to 800 mg, for instance from 100 to 200 mg divided into 1 to 10, preferably into 1 to 2 individual doses. The daily dose of safinamide is from 10 to 600 mg, for instance from 50 to 150 mg divided into 1 to 10, preferably into 1 to 2 individual doses.

The invention will be further clarified by the following non-limiting examples.

### EXAMPLES

### Example 1

| | **Composition 1** | **Composition 2** | **Composition 3** | **Composition 4** |
|---|---|---|---|---|
| Entacapone | 200.0 | 200.0 | 200.0 | 200.0 |
| Croscarmellose sodium | 100.0 | 87.0 | 87.0 | 100.0 |
| Microcrystalline cellulose | 20.0 | 73.0 | 58.0 | 40.0 |
| Mannitol | 20.0 | - | - | - |
| Maize starch | 20.0 | - | - | - |
| Povidone | - | - | 19.0 | 17.0 |
| Hydroxypropyl methylcelulose 6 cps | 20.0 | 20.0 | - | - |
| Purified water | q.s. | q.s. | q.s. | q.s. |
| Magnesium stearate | 7.0 | 6.0 | 6.0 | 7.0 |
| Film-coating | 15.0 | 15.0 | 15.0 | 15.0 |
| Theoretical weight of tablet | 402.0 | 401.0 | 385.0 | 379.0 |

Entacapone having at least 90 % of the particles less than 30 µm and not more than 20 % of the particles less than 3 µm was obtained by mulling with a fluid energy mill (an opposed jet type with a dynamic classifier).

Entacapone, croscarmellose sodium (distributed by AWL Scandinavia AB under trademark Ac-Di-Sol®), microcrystalline cellulose (distributed by AWL Scandinavia AB) and povidone (distributed by BASF Aktiengesellschaft under trademark Kollidon® K 30) or hydroxypropyl methylcellulose (distributed by Syntapharm GmbH) and possible mannitol (distributed by Roquette Freres) and maize starch (distributed by Cerestar Scandinavia A/S) are mixed in a high-shear mixer (Diosna P 25) for 1 min. Thereafter, the granulation liquid (purified water) is sprayed to the pre-mixed powder blend.

The granules are dried in a fluid-bed granulator (Glatt WSG 5) and screened with a conical mill (Glatt GS 100, mesh size of friction screen 1.3 mm).

The mass is finalized by adding magnesium stearate (distributed by Mallinckrodt Chemical Limited) to the granules and mixing for 5 min (Turbula T10B).

Tablets are compressed with a rotary tablet press (Fette P I) by using compression force of approx 7 kN and tabletting speed of approx. 16 000 tablets/h. Core tablets are coated with a PVA-based film (distributed by Colorcon under trademark Opadry®) in a coating drum (Accela-Cota 24").

With composition 1, tablets with good technical properties (high crushing strength, low weight variation, low friability, no observed sticking during tablet compression) are achieved.

Especially good quality to the product can be achieved with compositions 2 to 4, with which the tablet disintegration and flowability of the tabletting mass can be improved. Increased flowability decreases the weight variation of the tablets, which can be seen in improved content uniformity. Also, no lamination is observed with said formulations in spite of the high amount of croscarmellose sodium.

### Example 2

| | **Composition 5** | **Composition 6** | **Composition 7** |
|---|---|---|---|
| Entacapone | 200.0 | 200.0 | 200.0 |
| L-HPC, LH-21 | 90.0 | 60.0 | 90.0 |
| Croscarmellose sodium | - | 50.0 | - |
| Microcrystalline cellulose | - | 30.0 | - |
| Mannitol | - | 20.0 | - |
| Povidone | 14.0 *) | 12.0 *) | 17.0 **) |
| Purified water | q.s. | q.s. | q.s. |
| Colloidal silica | - | - | 2.0 |
| Magnesium stearate | 6.0 | 5.0 | 6.0 |
| Film-coating | 15.0 | 15.0 | 15.0 |
| Theoretical weight of tablet | 325.0 | 392.0 | 330.0 |

| | | | |
|---|---|---|---|
| *) Binder dissolved in purified water **) Binder added as a powder | | | |

Entacapone, low-substituted hyroxypropylcellulose (L-HPC, distributed by Syntapharm GmbH and possible croscarmellose sodium (distributed by AWL Scandinavia AB under trademark Ac-Di-Sol®), microcrystalline cellulose (distributed by AWL Scandinavia AB) and mannitol (distributed by Roquette Freres), as well as povidone (distributed by BASF Aktiengesellschaft under trademark Kollidon® K 30) in case of composition 5, are pre-mixed in a high-shear mixer (Diosna P 25) for 1 min.

Povidone is dissolved in purified water (compositions 5 and 6) forming a solution with concentration of approx. 6 % w/w. The granulation liquid (purified water or povidone solution) is sprayed to the pre-mixed powder blend.

Granules are dried in a drying chamber (Memmert), or most preferably in a single pot processor and screened with a conical mill (Glatt GS 100, mesh size of friction screen 1.3 mm). The mass is finalized by adding magnesium stearate (distributed by Mallinckrodt Chemical Limited) and possible colloidal silica (distributed by Algol Chemicals Oy under trademark Aerosil® 200) to the granules and mixing for 5 min (Turbula T10B).

Tablet compression is performed with a rotary tablet press (Fette P 1) with compression force of approx. 14 kN and tabletting speed of approx. 16 500 tablets/h.

Core tablets are coated with a PVA-based film (distributed by Colorcon under trademark Opadry®) in a coating drum (Accela-Cota 24").

Tablets with good technical properties are achieved with composition 5 described above. The flowability of the mass, and thus the processability of the product, can be improved with extra-granular colloidal silica (composition 7). Even better flowability can be achieved with composition 6.

### Example 3

| | **Composition 8** | **Composition 9** | **Composition 10** |
|---|---|---|---|
| Entacapone | 200.0 | 200.0 | 200.0 |
| **Intragranular excipients** | | | |
| Croscarmellose sodium | 80.0 | 80.0 | 45.0 |
| Microcrystalline cellulose | 30.0 | 30.0 | - |
| Polyethylene glycol 3000 | - | - | 105 |
| Povidone | 15.0 | 15.0 | - |
| Purified water | q.s. | q.s | - |
| **Extragranular excipients** | | | |
| Maize starch | q.s. | q.s | - |
| Sodium starch glycolate | - | 8.0 | - |
| Magnesium stearate | 5.0 | - | - |

### Compositions 8 and 9

Entacapone, croscarmellose sodium (distributed by AWL Scandinavia AB under trademark Ac-Di-Sol®), microcrystalline cellulose (distributed by AWL Scandinavia AB) and povidone (distributed by BASF Aktiengesellschaft under trademark Kolhdon® K 30) are mixed in a high-shear mixer (Diosna P 25) for 1 min. Thereafter, the granulation liquid (purified water) is sprayed to the pre-mixed powder blend.

The granules are dried in a fluid-bed granulator (Glatt WSG 5) and screened with a conical mill (Glatt GS 100, mesh size of friction screen 1.3 mm).

The mass is finalized by adding a proper amount of maize starch (distributed by Cerestar Scandinavia A/S) to adjust the volume of the mass to fit to capsule shell no. 0 and magnesium stearate (distributed by Mallinckrodt Chemical Limited) or sodium starch glycolate (a disintegrant), respectively, to the granules and mixing for 5 min (Turbula T10B).

Capsules are filled using volumetric tray filling (Chem. & Pharm. Ind. Co., Inc.).

### Composition 10

Entacapone and PEG 3000 are mixed with a spatula in a decanter. Thereafter the mixture is hot melt granulated on a heating plate. The granules are screened through a 1.5 mm screen. Capsule shells No. 0 are filled with granules by weight.

### Example 4

| | **Composition 12** | **Composition 13** | **Composition 14** | **Composition 15** |
|---|---|---|---|---|
| Entacapone | 200 | 200 | 200 | 200 |
| Crospovidone | 42 | - | - | - |
| Sodium starch glycolate | - | 53 | - | - |
| Carboxymethylcellulose calcium | - | - | 49 | - |
| Polacrilin potassium | - | - | - | 51 |
| Microcrystalline cellulose | 94 | 36 | 77 | 66 |
| Mannitol | 17 | - | - | - |
| Pregelatinized starch | - | 65 | 51 | 45 |
| Povidone | - | 21 | - | - |
| Hydroxypropyl methylcelulose 6 cps | - | - | 19 | - |
| Hydroxypropyl celulose | 18 | - | - | 18 |
| Purified water | q,s, | q,s, | q,s, | q,s, |
| Magnesium stearate | 5 | 6 | 5 | 5 |
| Film-coating | 15 | 15 | 16 | 16 |
| Theoretical weight of tablet | 391 | 396 | 417 | 401 |

### Compositions 12 to 15

Entacapone and other intragranul excipients, respectively, are mixed in a high-shear mixer (Diosna P 25) for 1 min. Thereafter, the granulation liquid (purified water) is sprayed to the pre-mixed powder blend.

The granules are dried in a fluid-bed granulator (Glatt WSG 5) and screened with a conical mill (Glatt GS 100, mesh size of friction screen 1.3 mm).

The mass is finalized by adding magnesium stearate (distributed by Mallinckrodt Chemical Limited) to the granules and mixing for 5 min (Turbula T10B).

Tablets are compressed with a rotary tablet press (Fette P I) by using compression force of approx 13 kN and tabletting speed of approx. 16 000 tablets/h. Core tablets are coated with a PVA-based film (distributed by Colorcon under trademark Opadr®) in a coating drum (Accela-Cota 24").

## Claims

1. An oral dosage form of entacapone allowing at least 50 % of entacapone to be released within the first 30 minutes in a dissolution test (Apparatus 2 (USP), paddles at 50 rpm, pH 5.5) from said dosage form and comprising granules made by a method comprising (i) preparing a mixture of entacapone as the sole drug substance with a binder and optionally one or more other excipients and granulating the mixture with a granulating liquid, or granulating entacapone together with one or more optional other excipients with a granulating liquid comprising a binder, or by melt granulation; (ii) drying or cooling, respectively and optionally screening the granules of step (i).

2. The dosage form of claim 1, wherein the entacapone used in the preparation of the granules is in particulate form and at least 90% of entacapone particles have a diameter less than 55 µm.

3. The dosage form of claim 1, wherein the entacapone used in the preparation of the granules is in particulate form and at least 90% of entacapone particles have a diameter less than 35 µm.

4. The dosage of any of the preceding claims, wherein the entacapone used in the preparation of the granules is in particulate form and not more than 20% of entacapone particles have a diameter less than 2 µm.

5. The dosage of any of the preceding claims, wherein the binder is one or more of acacia, alginic acid, carbomer, carboxymethylcellulose sodium, ceratonia, cottonseed oil, dextrin, dextrose, gelatin, guar gum, hydrogenated vegetable oil type I, hydroxyethyl cellulose, hydroxyethylmethyl cellulose, hydroxypropyl cellulose, low substituted hydroxypropyl cellulose, hypromellose, magnesium aluminium silicate, maltodextrin, maltose, methylcellulose, microcrystalline cellulose, polydextrose, polyethylene oxide, polymethacrylates, povidone, sodium alginate, starch, pregelatined starch, stearic acid, sucrose and zein.

6. The dosage of any of the preceding claims, wherein the binder is one or more of povidone, hypromellose, hydroxypropyl cellulose, methylcellulose and gelatine.

7. The dosage of any of the preceding claims, wherein the granulating liquid is water, a lower alcohol or a mixture thereof.

8. The dosage of any of the preceding claims, wherein one or more other excipients is present and comprises a disintegrant.

9. The dosage form according to claim 8, wherein the disintegrant is one or more of alginic acid, calcium phosphate, tribasic, carboxymethylcellulose calcium, carboxymethylcellulose sodium, colloidal silicon dioxide, croscarmellose sodium, crospovidone, docusate sodium, guar gum, low substituted hydroxypropyl cellulose, magnesium aluminun silicate, methylcellulose, microcrystalline cellulose, povidone, sodium alginate, sodium starch glycolate, polacrilin potassium, silicified microcrystalline cellulose, starch and pre-gelatinezed starch.

10. The dosage form according to claim 8, wherein the disintegrant is one or more of croscarmellose sodium, crospovidone, low substituted hydroxypropyl cellulose, microcrystalline cellulose and sodium starch glycolate.

11. The dosage form according to claim 8, wherein the disintegrant is croscarmellose sodium and the granulation liquid is water, and the amount of water added during the granulation is from 50% to 120% compared with the dry weight of the granulation mass.

12. The dosage form according to claim 8, wherein the disintegrant is L-HPC and the amount of water added during granulation is from 30% to 100% compared with the dry weight of the granulation mass.

13. The dosage form according to any of the preceding claims, wherein the granules are screened to a granule size distribution by volume, in which the 10% fractile is from 7 µm to 60 µm, the 90% fractile is from 600 µm to 1200 µm, and the median is from 80 µm to 600 µm.

14. The dosage form according to any of the preceding claims, wherein the granules are screened to a granule size distribution by volume, in which the 10% fractile is from 10 µm to 40 µm, the 90% fractile is from 750 µm to 1100 µm, and the median is from 100 µm to 300 µm.

15. The dosage form of any one of the preceding claims, wherein the granules comprise polymorph A of the (E) isomer of entacapone or polymorph D of the (E) isomer of entacapone.
